# EUROPEAN PATENT APPLICATION

(11) **EP 2 517 716 A1**
(43) Date of publication of application: **31.10.2012**
(21) Application number: 12178294.0
(22) Date of filing: 29.09.2008
(51) Int. Cl.: A61K 36/15, A61K 36/18, A61K 36/23, A61K 36/28, A61K 36/324, A61K 36/35, A61K 36/45, A61K 36/53, A61K 36/537, A61K 36/539, A61K 36/67, A61K 36/77, A61K 36/82, A61K 36/9066, A61P 31/02

(54) **Oral compositions containing botanical extracts**

(30) Priority: 01.10.2007 US 976508 P
(62) Divisional of application: 08835291.9
(71) Applicant: Colgate-Palmolive Company, New York NY 10022-7499 (US)
(72) Inventor: Cummins, Diane, Linvingston, NJ New Jersey 07039 (US); Trivedi, Harsh M, Hillsborough, NJ New Jersey 08844 (US)
(74) Representative: Jenkins, Peter David

(57) **Abstract**

The disclosure provides oral compositions having at least two botanical active ingredients derived from plants. The oral composition also includes an orally acceptable vehicle to deliver an effective amount of the at least two active ingredients *in vivo.* The botanical active ingredients provide particularly efficacious antimicrobial (antibacterial, antiviral, and/or antifungal), antioxidant, anti-inflammatory, anti-ageing, and or healing properties to the oral compositions.

## Description

### BACKGROUND OF THE INVENTION

Oral inflammation is associated with common oral conditions, including periodontitis, for example. Gingivitis is the initial stage of gum disease. A cause of gingivitis is plaque, which is a soft, sticky, colorless film of bacteria that forms on the teeth and gums. Plaque, if left untreated, produces toxins that can inflame or infect the gum tissue to cause gingivitis. Untreated gingivitis can eventually spread from the gums to the ligaments and bone that support the teeth, and can cause periodontitis.

A wide variety of antibacterial agents have been suggested in the art to retard plaque formation and the oral infections associated with plaque formation. It is difficult to predict the antiplaque efficacy of antibacterial compounds when incorporated into an oral care composition with other active ingredients. Further, many antibacterial agents negatively interact with one or more components in the oral care delivery vehicle so that effective performance of such oral compositions is diminished, including toothpaste and mouthrinse. Notwithstanding the efficacy of certain antibacterial agents, there is a continuing interest to develop oral care compositions which improve the efficacy and/or bioavailability of oral care compositions *in vivo.* Further, an oral care composition having multiple efficacies in the oral cavity, for example, combating plaque, gingivitis, periodontitis or diseases of the oral cavity, while further having other effects, such as anti-inflammatory effects, are desirable. Additionally, oral compositions that contain natural or botanically-based active ingredients are desirable.

### BRIEF SUMMARY OF THE INVENTION

In various aspects, the disclosure provides oral compositions having at least two botanical active ingredients derived from plants. The oral composition also includes an orally acceptable vehicle to deliver an effective amount of the at least two active ingredients *in vivo.* The oral compositions provide antimicrobial (antibacterial, antiviral, anti-and/or antifungal), antioxidant, anti-inflammatory, anti-ageing, and or healing properties.

In various aspects, an oral composition includes at least two botanical active ingredients chosen from one or more plants of the following the genera: *Origanum Thymus, Lavandula, Salvia, Melissa, Cuminum, Petroselinum, Calendula, Tagetes, Boswellia, Sambucus, Copaifera, Curcuma, Allium, Symphytum, Punica, Euterpe, Sophora, Rheum, Fagopyrum, Camellia, Coptis, Hydrastis, Mahonia, Phellodendron, Berberis, Xanthorhiza, Lonicera, Vaccinium, Cinnamomum, Vitis, Terminalia, Pinus, Albizia, Melia, Salvadora, Paullinia, Piper, Syzygium, Commiphora, Juglans, Scutellaria, and Magnolia;* and an orally acceptable vehicle to deliver an effective amount of the at least two active ingredients *in vivo.*

In yet another aspect, the oral composition includes at least two botanical active ingredients chosen from one or more plants of the following species: *Origanum vulgare, Origanum onites, Origanum majorana, Origanum heracleoticum, Thymus vulgaris L, Thymus citriodorus, Thymus pulegioides, Thymus x herba-barona, Thymus serpyllum, Lavandula angustifolia*/*officinalis, Lavandula stoechas, Lavandula dentate, Lavandula x intermedia, Lavandula multifida, Salvia officinalis, Salvia divinorum, Salvia apiana, Melissa officinalis, Cuminum cyminum, Petroselinum crispum, Calendula arvensis, Calendula maderensis, Calendula officinalis, Tagetes erecta, Tagetes minuta, Tagetes patula, Boswellia sacra, Boswellia frereana, Boswellia serrata, Boswellia papyrifera, Sambucus nigra, Sambucus melanocarpa, Sambucus racemosa, Copaifera langsdorfii, Curcuma longa, Allium sativu, Symphytum officinale, Punica granatum, Euterpe oleracea, Sophora flavescens, Rheum rhabarbarum, Rheum rhaponticum, Fagopyrum esculentum, Camellia sinensis, Coptis teeta, Hydrastis canadensis, Mahonia aquifolium, Phellodendron amurense, Berberis vulgaris, Xanthorhiza simplicissima, Lonicera ceprifoliu, Vaccinium macrocarpon, Cinnamomum zeylanicum Nees, Cinnamomum verum, Vitis Vinifera, Terminalia Bellerica, Pinus Pinaster, Albizia Lebbek, Melia Azadirachta, Salvadora persica, Paullinia cupana, Piper betle, Syzygium aromaticum, Commiphora myrrha, Juglans regia, Scutellaria baicalensis, and Magnolia officinalis.*

Further uses, benefits and embodiments of the present invention are apparent from the description set forth herein. It should be understood that the description and specific examples are intended for purposes of illustration only and are not intended to limit the scope of the present disclosure.

### DETAILED DESCRIPTION OF THE INVENTION

As used throughout, ranges are used as shorthand for describing each and every value that is within the range. Any value within the range can be selected as the terminus of the range.

Suitable botanical active ingredients for use in the oral compositions can include natural extracts or active compounds derived from natural sources or compounds. As referred to herein, an "extract" suitable for use in the various embodiments of the disclosure can be obtained from any part of a plant including the leaf, stem, stalk, cortex (*i.e.,* bark), pulp, seed, flesh, juice, root, flower, or any other suitable part of a plant or other natural source. The term "botanical active ingredient" encompasses extracts, oils or galenical compositions, active compounds, derivatives, synthetic or semi-synthetic equivalents of such natural extracts and/or active compounds contained therein. Thus, in certain aspects, one or more of the active ingredients includes a derivative or synthetic compound similar to the compounds (thus "derived from") from the natural sources, such as natural botanical extracts. It should be noted that certain natural extracts are in lipophilic carriers, such as is the case with essential oils, or where the extract is diluted in an oil carrier. Other extracts may be partially or fully separated from the lipophilic carriers and merely contain the active compounds of the extract and hydrophobic carriers or solvents. The extracts may be in liquid or dried powder forms.

As used herein, "extracting" or "extraction" of a solid or liquid material means contacting the material with an appropriate solvent to remove the substance(s) desired to be extracted from the material. Where the material is solid, it is preferably dried and crushed or ground prior to contacting it with the solvent. Such an extraction may be carried out by conventional means known to one of skill in the art, for example, by using an extraction apparatus, such as a Soxhlet apparatus, which retains the solid material in a holder and allows the solvent to flow through the material; by blending the solvent and material together and then separating the liquid and solid phases or two immiscible liquid phases, such as by filtration or by settling and decanting. In various embodiments, the botanical active ingredients used in oral care compositions are of reproducible, stable quality and have microbiological safety.

In various aspects, combinations of two or more botanical active ingredients may provide benefits for an oral care composition, enhancing the antimicrobial, anti-plaque, anti-gingivitis, anti-periodontitis, anti-calculus, anti-inflammatory, anti-oxidant, anti-ageing, and/or healing effects of the oral composition. In various aspects, certain specific combinations of botanical active ingredients are particularly beneficial by enhancing the efficacy of other oral care active ingredients, whether botanical or non-botanical. Many extracts have a large number of active compounds, which represent a wide complement that contributes to efficacy in a variety of areas and functionality. The inclusion of two or more extracts as botanical active ingredients provides additional complementary, and in some cases, unexpected benefits when used in combination with one another. The compositions of the invention may also be used to ameliorate and/or maintain systemic health.

The botanical active ingredients that are useful in the oral compositions are preferably safe and suitable for use in mammals. In various embodiments, the oral compositions of the present disclosure comprise about 0.0001% to about 10%, preferably about 0.001% to about 5%, more preferably about 0.01% to about 3% of a cumulative amount of the botanical active ingredients based on a total amount of the oral composition. (As used herein, all percentages are by weight % of the total composition weight, unless otherwise indicated.)

Additionally, the concentration of botanical active ingredients in the oral care composition depends upon the relative concentration of the active compounds in the extract and the required dosing for bioavailability, and as such, it is contemplated that the amount of botanical active ingredients present may vary as recognized by one of skill in the art. In various aspects, the oral compositions have an amount of the two or more botanical active ingredients so that the amount delivered to the oral cavity upon use is effective to provide the desired effects. Alternatively, the composition may contain three, four, five, six, seven, eight, nine, ten, eleven, twelve, thirteen, fourteen, fifteen, sixteen, seventeen or more. Additionally, the concentration of the botanical active ingredients is typically dependent upon the form of the oral composition. For example, mouthrinses typically have a relatively low concentration of an active ingredient, as where dentifrices, gels, or toothpowders have a higher concentration to achieve the same delivered dosage based on ease of dispersion. Likewise, confectionary compositions typically have a relatively high concentration of active ingredient to enable sufficient dispersion as they dissolve or are masticated.

The following description pertains to suitable plant sources from which the botanical active ingredients can be derived for use in an oral composition. As will be described in more detail below, combinations of more than two natural active ingredients are feasible and in some aspects, highly desirable. As discussed below, the botanical active ingredients provide one or more of the following benefits in an oral care composition: antimicrobial (antibacterial, antiviral, and/or antifungal), antioxidant, anti-inflammatory, anti-ageing, and or healing properties.

For example, benefits of certain botanical active ingredients include collagenase inhibition and/or sirtuins activation. Cytokines are activated by a mammal's immune system response, which can induce collagenase production by stimulating cells, such as fibroblasts & osteoblasts, thus resulting in indirect tissue damage. Thus, the botanical active ingredient may minimize collagenase activity or production. On the other hand, in some aspects, the botanical active ingredients may increase sirtuins enzyme activity (*e.g.*, Sir2), which are hypothesized to be involved in the body's response to stress conditions and to enhance lifespan-extending effects, thus promoting anti-ageing.

As discussed herein, the two or more botanical active ingredients are derived from or based upon compounds or extracts isolated from plants. The following plants each provide one or more active ingredients that are useful in an oral composition for one or more oral care benefits. For example, extract from *Romains officinalis* (rosemary) has an antibacterial and anti-inflammatory effect. Rosemary extract contains various organic and inorganic materials, including flavonoids, triterpenic and phenolic acids. Non-limiting examples of the useful organic compounds include 1,8-cineole, camphor, a-pinene, carnosic acid, rosmarinic acid, ursolic acid, carnosol, and oleanolic acid. The discussion of active compounds contained herein in relation to various extracts includes those compounds that are believed to be efficacious in oral compositions; however, the lists of such compounds are non-exclusive and in some cases are yet to be identified or fully characterized, however, empirical observation demonstrates the desired effects. Furthermore, in various aspects, the entire extract including all compounds contained therein provides the most effective botanical active ingredient. Rosemary extracts for use in oral compositions are discussed in U.S. Patent Publication 2006/0134025 to Trivedi et al. and assigned to Colgate-Palmolive, which is herein incorporated by reference in its entirety. Each additional citation to a reference contained in the description is expressly incorporated by reference in its entirety. The extracts of the leaves of rosemary plants are sold as rosemary extract by, for example, Sabinsa Corporation of Piscataway, New Jersey. Such compounds found in various plant-based extracts may be isolated from the extracts and used independently as botanical active ingredients. For example, carnosic acid may be independently isolated and used in an oral composition, as it has been found to be efficacious against oral bacteria that cause cavities, gingivitis, and bad breath.

Other extracts useful in accordance with the present teachings include any suitable part of a plant from the Lamiaceae family, including those plants classified in the following genera: *Origanum, Thymus, Lavandula, Salvia, Perovskia, Phlomis,* or *Melissa.* For example, suitable extracts include those from *Origanum vulgare L.* (commonly known as "oregano", "wild oregano", or "wild marjoram"), including its sub-species (*Origanum vulgare* ssp.), *Origanum onites* (commonly known as "Italian oregano" or "pot marjoram"), *Origanum majorana* (commonly known as "marjoram" or "sweet marjoram") and *Origanum heracleoticum. Origanum vulgare* subspecies include *O. vulgare ssp. vulgare, O. vulgare ssp. viride,* and *O. vulgare ssp. hirtum* (commonly known as "Greek oregano" or "Wild oregano"). "Oregano" encompasses all suitable species and sub-species of the genus Origanum. Oregano is believed to contain over 30 active compounds, including carvarcrol, thymol, and rosmarinic acid.

The genus *Thymus* (Thyme), also of the family Lamiaceae, includes over three hundred species and sub-species. Suitable extracts include those isolated from the following plants: *Thymus vulgaris L, T citriodorus, T. pulegioides, T. x herba-barona, T. serpyllum.* "Thyme" encompasses all suitable species and sub-species of the genus *Thymus,* and extracts derived therefrom, which are believed to contain carvarcrol and thymol active compounds. Other suitable extracts include those from the *Lavandula* (lavender) genus, which encompasses over 30 species. Suitable lavender species include *Lavandula angustifolia* (formerly known as *L. Officinalis L.*)*, L. stoechas; L. dentate; L. x intermedia;* and *L. multifida.* Lavender extracts contain the active compounds linalyl acetate and linalool, among others. "Sage" generally includes plants of three genera of the Lamiaceae family, namely *Salvia, Perovski,* and *Phlomis.* In certain aspects, useful plants include *Salvia officinalis* (common sage), *S. divinorum* (diviner's sage); and *S. apiana* (white sage). Extracts from *S. officinalis* have antibiotic, antifungal, and astringent effects, among others, Another suitable extract is derived from the lemon balm plant (*Melissa Officinalis*), which has antibacterial and antiviral properties.

Further extracts useful in accordance with the present teachings also include those derived from plants of the Apiaceae family, including *Cuminum* and *Petroselinum. Cuminum cyminum* (Cumin) contains various active compounds, including cuminaldehyde and pyrazines. *Petroselinum crispum* (parsley) includes apiol, furanocourmarin, and psoralen compounds. Cumin and parsley extracts have beneficial antioxidant activity, *inter alia.*

Genera *Calendula* and *Tagetes,* both commonly known as "marigold," are both of the family Asteraceae. The *Calendula* genus include many species and sub-species, including *Calendula arvensis* (field marigold); *C*. *maderensis* (Madeiran marigold); and *C*. *officinalis* (pot marigold). *Calendula* extracts contain various active compounds, including calendic acid. The Tagetes genus includes over sixty species and sub-species, including *Tagetes erecta; T. minuta, T. patula* and the like. Extracts of both *Calendula* and *Tagetes* have antioxidant and anti-inflammatory activity and are efficacious against oral bacteria that cause cavities, gingivitis and bad breath.

Boswellia is a genus of trees that produce extracts having anti-inflammatory properties, including boswellic acid compounds. For example, *Boswellia sacra, B. frereana; B. serrata;* and *B. papyrifera* and their sub-species produce suitable extracts. A useful active compound isolated from the Boswellia plant is acetyl keto β-boswellic acid (AKBBA), for example, 3-acetyl 11-keto β-boswellic acid, which exhibits antibacterial, anti-inflammatory and antioxidant activities. A commercially available *B. serrata* extract including a mixture of β-boswellic and organic acids is available from Sabinsa Corp., as BOSWELLIN^{™} CG.

Sambucus includes over thirty species and subspecies, which are commonly referred to as elderberry or elder. Various *Sambucus* species are suitable, including *Sambucus nigra* (common elder); *S. melanocarpa* (blackberry elder); *S. racemosa* (red-berried elder), among others. The elderberry extracts have been discovered to have antioxidant activity, and further, provide one or more of the following benefits in an oral composition: antibacterial, antioxidant, collagenase inhibition, sirtuins activation, and anti-inflammatory properties.

Extracts of *Copaifera langsdorfii* (copaiba balsam) are useful, as are *Curcuma longa* (tumeric), which includes the compounds curcumin, demethoxycurcumin, bis-demethoxycurcumin, and tetrahydrocurcuminoid. Additional suitable extracts include those isolated from *Allium sativum* (garlic) or other plants of the *Allium* genera. Garlic extracts contain allicin, alliin, ajoene, and other flavonoids, which provide antioxidant and/or anti-microbial benefits. Extracts from *Symphytum officinale* (comfrey) or other plants of the genus *Symphytum* are useful as anti-oxidants, anti-inflammatory, and/or antimicrobial agents; as are *Punica granatum* (pomegranate) extracts which include various antioxidant polyphenols, such as hydrolyzable tannins punicalagins; *Euterpe oleracea* (Açaí palm), which contains resveratrol, anthocyanins, and various other flavonoid and flavonoid-like compounds, such as homoorientin, orientin, tasifolin, deoxyhexose, isovitexin, scoparin; *Sophora flavescens* extracts, which contain kurarinone as a bioactive flavonoid, which has anti-inflammatory and antibacterial function. Each of the extracts described above exhibits one or more antioxidant, anti-inflammatory, antiviral, and/or antibacterial properties. A representative structure of kurarinone is:

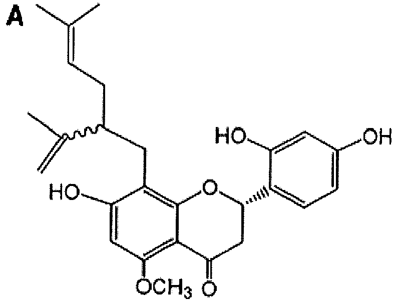

In certain aspects of the disclosure, the oral compositions optionally comprise a commercially available extract derived from *C*. *longa* that includes tetrahydrocurcuminoid, under the trade name SABIWHITE^{™} available from Sabinsa Corp., which is believed to have the following representative structure:

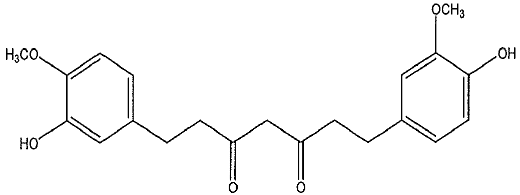

Various plant extracts contain the active compound rutin (quercetin-3-rutinoside) which is an antioxidant flavonoid glycoside (comprising the flavonol quercetin and the disaccharide rutinose) found in various plants of the Polygonaceae family, including the Rheum genus, including *Rheum rhabarbarum* and *R*. *rhaponticum* (garden rhubarb) and of the *Fagopyrum esculentum* Moench (buckwheat) plant. What is believed to be a representative structure is shown below:

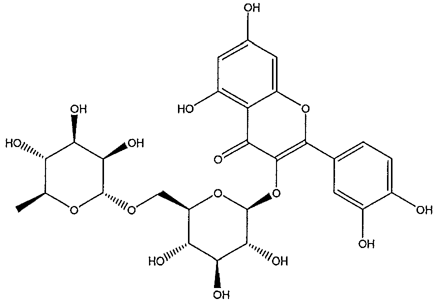

Rutin is believed to scavenge superoxide radicals, chelate metal ions, modulate bursts of neturophils, inhibit lipid peroxidation, maintain the biological antioxidant reduced glutathione, and has involvement in fenton reactions (which generate reactive oxygen species). Thus, rutin has antioxidant, anti-inflammatory, anticarcinogenic, antithrombotic, cytoprotective and vasoprotective activities, which are beneficial for oral compositions. Further, rutin augments antiplaque and antioxidant activity in oral compositions.

Non-limiting examples of antibacterial, antioxidant, and/or anti-inflammatory natural extracts include those isolated from green or oolong tea, cinnamon, gold thread, cranberry and other *Ericaceae* family plants, honeysuckle, grape seed, myrobalan, rosemary, east Indian walnut, neem, niruri, and pine bark.

Green tea and oolong tea are isolated from the *Camellia sinensis.* Any variety, form, or subspecies of *Camellia sinensis* may be used and these may be selected from any subspecific taxon thereof, suitable examples of which are: *C. sinensis var. assamica,* which includes, *e.g.*, the former *C*. *assamica and var. kucha; C. sinensis var. cambodiensis,* which includes, e.g., the former *subsp. lasiocalyx and var. Shan; C. sinensis var. dehungensis; C. sinensis var, pubilimba; and C. sinensis var. sinensis,* which includes, *e.g.*, the former *vars. bohea, macrophylla, parvifolia,* and *waldenae.* The active components of *Camellia sinensis* extracts are believed to be the polyphenol catechines including catechin, epocatechin, epigallocatechin, epicatchin gallate, gallocatechin and epigallocatechin. Extracts of unoxidized camellia (*e.g.*, green tea) used in oral compositions are described in U.S. Patent Publication No. 2006/0141073 to Worrell and extracts of oxidized camellia (*e.g.,* oolong tea) are in U.S. Patent Publication No. 2006/0141039 to Boyd, et al., both assigned to Colgate-Palmolive. An example of a suitable Camellia extract is "Green Tea Extract CG," specification no. MS-0726-01, available from Sabinsa Corp.

Gold thread extracts are obtained from one or more of the following plant families *Annonaceae, Berberidaceae, Menispermaceae, Papaveraceae, Ranunculaceae, Rutaceae, Zingiberaceae, Nadina, Mahonia, Thalictrum* spp. For example, a gold thread extract having desirable advantages in an oral care composition is *Coptis teeta* (coptis). The active compound of gold thread extracts is believed to be berberine (an anti-inflammatory, anti-microbial compound). Goldenseal (Orange-root), *Hydrastis canadensis,* is of the family *Ranunculaceae,* and one of its active components is believed to be berberine, as well as hydrastine alkaloids. Other extracts having berberine as an active compound *include Mahonia aquifolium* (Oregon grape), *Phellodendron amurense* (phellodendron), *Berberis vulgaris* (barberry), and *Xanthorhiza simplicissima* (yellow root).

Honeysuckle (*Lonicera ceprifolium*) extracts are obtained from the flower of the honeysuckle plant. The active polyphenol materials in the honeysuckle extract are believed to be the chlorogenic acid and/or lutenolin flavonoids. The *Ericaceae* family broadly refers to over 100 genera and the over 4,000 associated species, such as those disclosed in U.S. 5,980,869 to Sanker, et al. In certain embodiments, extracts from plants in the *Vaccinium* genus are useful as antibacterial natural extracts, such as cranberry (*Vaccinium macrocarpon*).

*Cinnamomum zeylanicum Nees* or *C*. *verum,* contain multiple active compounds including cinnamaldehyde, eugenol, ethyl cinnamate, beta-caryophyllene, linalool, and methyl chavicol. Extracts of cinnamon exhibit antioxidant and antibacterial activity. Grape seed or grape skin extracts are isolated from *Vitis Vinifera* plants and include various polyphenols, including resveratrol and antioxidant proanthocyanidins. Myrobalan is preferably extracted from *Terminalia Bellerica* fruit. Pine bark extract is preferably extracted from the cortex (bark) of *Pinus Pinaster* (Maritime pine), which includes pycnogenol and exhibits antibacterial, anti-inflammatory, antioxidant and anti-ageing activities. The extract of the cortex of the neem or margosa plant (*Melia Azadirachta*) is a known antibacterial component. Niruri or *Phyllanthus Niruri* extract is also a known antibacterial extract. *Salvadora persica* (miswak) extract provides efficacious antibacterial effects in oral care compositions. In certain aspects, a botanical active ingredient may be isolated from *Paullinia cupana* (guarana), whose extract includes caffeine, catechins, theobromine, theophylline and other alkaloids.

*Piper betle* (betel) extract, especially extract derived from betel leaves, includes active compounds including chavibetol, chavicol, estragole, eugenol, methyl eugenol, and hydroxy catechol; *Syzygium aromaticum* (clove) extracts have antiseptic and anesthetic properties and include the compounds eugenol, beta-caryophylline, vanillin, crategolic acid, methyl salicylate, tannins, flavanoids (including eugenin, kaempferol, rhamnetin, and eugentitin), triterpenoids (such as oleanolic acid, stigmasterol and campesterol), and various sesquiterpenes. *Commiphora myrrha* (myrrh) is likewise useful in oral compositions to provide antimicrobial and anti-inflammatory benefits. Another suitable genera of plants is *Juglans,* including *Juglans regia* (Persian walnut or common walnut tree) whose extract has anti-inflammatory and antioxidant properties. Similarly, the leaf of East Indian walnut (*Albizia Lebbek*) is suitable for use as an extract.

In certain aspects, the botanical active ingredient of the oral compositions comprises at least one free-B-ring flavonoid. Flavonoids are a group of compounds including such classes of compounds as flavones, flavans, flavonols, dihydroflanonols, flavonones, and derivatives thereof. Free-B-ring flavonoids active ingredients for use in oral compositions are described in U.S. Patent Publication No. 2006/0140881 to Xu et al. and assigned to Colgate-Palmolive.

In various embodiments, the botanical active ingredient comprises a free-B-ring flavonoid, which refers to a flavonoid compound that generally contains a 2,3-double bond and/or a 4-oxo group and lack any substituent groups on the aromatic B-ring. Such active ingredients for oral compositions are described in U.S. Patent Publication No. 2006/0140881 to Xu et al. and assigned to Colgate-Palmolive. Free-B-ring flavonoids can be isolated from plants of the family *Lamiaceae,* especially those of the subfamily *Scutellarioideae.* For example, the species *Scutellaria baicalensis* contains significant amounts of free-B-ring flavonoids, including baicalein, baicalin, wogonin, and baicalenoside. Free-B-ring flavonoids have antioxidant and anti-inflammatory properties and inhibit general activity of the cyclooxygenase enzyme COX-2. In certain aspects, the botanical active ingredients may optionally comprise either baicalin (also known by the Chinese name "Huangqingan"), 5,6-Dihydroxyflavone-7-O-glucoside, and baicalein (also known by the Chinese name "Huangqinsu"), 5,6,7-Trihydroxyflavone. In various embodiments, the botanical active ingredient of the oral compositions of the present disclosure may comprise baicalin, baicalein, or mixtures thereof.

Plants from the Magnoliaceae family, such as *Magnolia Officinalis* (magnolia) contain active compounds including: magnolol, honokiol, tetrahydromagnolol, and tetrahydrohonokiol, which have demonstrated bactericidal properties against various oral bacteria. In various aspects, either magnolol and/or honokiol are useful antibacterial botanical active ingredients. The use of active compounds from magnolia extract is described in U.S. Patent Publication Nos. 2006/0134024 to Trivedi et al. and 2006/0127329 to Xu et al., both assigned to Colgate-Palmolive.

Other suitable natural extracts that are known antimicrobial, antioxidant, and/or anti-inflammatory agents are those listed in the International Cosmetic Ingredient Dictionary and Handbook, Tenth Ed., 2004.

Treatment levels of the antibacterial components in various oral compositions are chosen to deliver an effective amount to the oral surfaces of the subject animal in which the oral compositions are applied. At lower treat levels, the antibacterial and other effects of the composition tend to be less significant. On the other hand, at the higher end of the treat level, increasing the level tends not to increase the effectiveness by a concomitant amount.

Thus, in various embodiments, at least two botanical active ingredients are derived from (either natural or synthetic products) one or more plants of the following the genera: *Origanum Thymus, Lavandula, Salvia, Melissa, Cuminum, Petroselinum, Calendula, Tagetes, Boswellia, Sambucus, Copaifera, Curcuma, Allium, Symphytum, Punica, Euterpe, Sophora, Rheum, Fagopyrum, Camellia, Coptis, Hydrastis, Mahonia, Phellodendron, Berberis, Xanthorhiza, Lonicera, Vaccinium, Cinnamomum, Vitis, Terminalia, Pinus, Albizia, Melia, Salvadora, Paullinia, Piper, Syzygium, Commiphora, Juglans, Scutellaria, and Magnolia.* The oral composition further comprises an orally acceptable vehicle to deliver an effective amount of the at least two active ingredients *in vivo,* which will be discussed in more detail below.

In certain aspects, at least two botanical active ingredients are derived from one or more plants of the following species: *Origanum vulgare, Origanum onites, Origanum majorana, Origanum heracleoticum, Thymus vulgaris L, Thymus citriodorus, Thymus pulegioides, Thymus x herba-barona, Thymus serpyllum, Lavandula angustifolia*/*officinalis, Lavandula stoechas, Lavandula dentate, Lavandula x intermedia, Lavandula multifida, Salvia officinalis, Salvia divinorum, Salvia apiana, Melissa Officinalis, Cuminum cyminum, Petroselinum crispum, Calendula arvensis, Calendula maderensis, Calendula officinalis, Tagetes erecta, Tagetes minuta, Tagetes patula, Boswellia sacra, Boswellia frereana, Boswellia serrata, Boswellia papyrifera, Sambucus nigra, Sambucus melanocarpa, Sambucus racemosa, Copaifera langsdorfii, Curcuma longa, Allium sativu, Symphytum officinale, Punica granatum, Euterpe oleracea, Sophora flavescens, Rheum rhabarbarum, Rheum rhaponticum, Fagopyrum esculentum, Camellia sinensis, Coptis teeta, Hydrastis canadensis, Mahonia aquifolium, Phellodendron amurense, Berberis vulgaris, Xanthorhiza simplicissima, Lonicera ceprifoliu, Vaccinium macrocarpon, Cinnamomum zeylanicum Nees, Cinnamomum verum, Vitis Vinifera, Terminalia Bellerica, Pinus Pinaster, Albizia Lebbek, Melia Azadirachta, Salvadora persica, Paullinia cupana, Piper betle, Syzygium aromaticum, Commiphora myrrha, Juglans regia, Scutellaria baicalensis, and Magnolia officinalis.*

In certain embodiments, the at least two botanical active ingredients are derived from one or more plants of the following species: *Romains officinalis, Origanum vulgare L, Thymus vulgaris L, Lavandula angustifolia*/*officinalis, Salvia officinalis, Melissa officinalis, Cuminum cyminum, Petroselinum crispum, Calendula officinalis, Tagetes erecta, Boswellia sacra, Sambucus nigra, Copaifera langsdorfii, Curcuma longa, Allium sativu, Symphytum officinale, Punica granatum, Euterpe oleracea, Sophora flavescens, Rheum rhabarbarum, Fagopyrum esculentum, Camellia sinensis, Coptis teeta, Hydrastis canadensis, Mahonia aquifolium, Phellodendron amurense, Berberis vulgaris, Xanthorhiza simplicissima, Lonicera ceprifoliu, Vaccinium macrocarpon, Cinnamomum zeylanicum Nees, Cinnamomum verum, Vitis Vinifera, Terminalia Bellerica, Pinus Pinaster, Albizia Lebbek, Melia Azadirachta, Salvadora persica, Paullinia cupana, Piper betle, Syzygium aromaticum, Commiphora myrrha, Juglans regia, Scutellaria baicalensis,* and *Magnolia officinalis.*

In certain embodiments, at least one of the two or more botanical active ingredients are derived from one or more plants of the following species: *Salvia officinalis, Salvia divinorum, Salvia apiana, Melissa Officinalis, Cuminum cyminum, Petroselinum crispum, Calendula arvensis, Calendula maderensis, Calendula officinalis, Tagetes erecta, Tagetes minuta, Tagetes patula, Boswellia sacra, Boswellia frereana, Boswellia serrata, Boswellia papyrifera, Sambucus nigra, Sambucus melanocarpa, Sambucus racemosa, Copaifera langsdorfii, Curcuma longa, Allium sativu, Symphytum officinale, Punica granatum, Euterpe oleracea, Sophora flavescens, Rheum rhabarbarum, Rheum rhaponticum, Fagopyrum esculentum, Coptis teeta, Hydrastis canadensis, Mahonia aquifolium, Phellodendron amurense, Berberis vulgaris, Xanthorhiza simplicissima, Lonicera ceprifoliu, Vitis Vinifera, Terminalia Bellerica, Pinus Pinaster, Albizia Lebbek, Melia Azadirachta, Salvadora persica, Paullinia cupana, Piper betle, Syzygium aromaticum, Commiphora myrrha, and Juglans regia.*

As discussed above, in certain embodiments at least one of the botanical active ingredients includes one or more compounds originating from one of the above identified plant species. By way of example, the following compounds are suitable for use, either individually or in combination, as a botanical active ingredient: 1,8-cineole, camphor, a-pinene, carnosic acid, rosmarinic acid, ursolic acid, and/or oleanolic acid (from rosemary); carvarcrol, thymol, and/or rosmarinic acid (from oregano or thyme); linalyl acetate and linalool (from lavender); cuminaldehyde, pyrazine, and/or tetrahydrocurcuminoid (from cumin); apiol, furanocourmarin, and/or psoralen (from parsley); calendic acid (from *Calendula* or *Tagetes* marigolds); boswellic acid, acetyl keto beta-boswellic acic (AKBBA), and/or 3-acetyl 11-keto β-boswellic acid (from boswellia); curcumin, demethoxycurcumin, bis-demethoxycurcumin, and/or other tetrahydrocurcuminoid (tumeric); allicin, alliin, ajoene, and/or other flavonoids (from garlic); resveratrol, anthocyanins, homoorientin, orientin, tasifolin, deoxyhexose, isovitexin, and/or scoparin (Açaí palm tree); kurarinone (from *Sophora flavescens*); rutin (from buckwheat or rhubarb); catechin, epocatechin, epigallocatechin, epicatchin gallate, gallocatechin and/or epigallocatechin (from green or oolong tea); berberine (from gold thread, golden seal, Oregon grape, barberry, phellodendron, barbarry, or yellow root); cinnamaldehyde, eugenol, ethyl cinnamate, beta-caryophyllene, linalool, and/or methyl chavicol (from cinnamon); resveratrol, proanthocyanidins, and/or polyphenols (from grapes); myrobalan (from *Terminalia Bellerica*); caffeine, catechins, theobromine, theophylline and/or other alkaloids (from guarana); havibetol, chavicol, estragole, eugenol, methyl eugenol, and/or hydroxy catechol (from betel).

In certain embodiments, the botanical active ingredients comprise an active ingredient selected from each of the plants: *Origanum vulgare L*; *Thymus vulgaris L*; *Romains officinalis L., Lavandula angustifolia*/*officinalis L.*; and *Hydrastis canadaensis L*.; and optionally *Cinnamonmum zeylanicum Nees.* Such a botanical active ingredient, including the extracts of oregano, thyme, rosemary, lavender, and golden seal, and optionally cinnamon is described in U.S. Patent Publication No. 2004/0213861 to D'Amelio, Sr. et al. This mixture of botanical or plant materials and extracts containing active compounds are combined in a manner to provide antimicrobial activity. With the exception of *Hydrastis canadensis,* each of the botanical materials are optionally present in an amount of about 5 wt % to about 40 wt % based on the total weight of the antimicrobial botanical mixture. Due primarily to its limited solubility, *Hydrastis canadensis* is included in amounts of 0.1 wt % or less, and typically 0.01 wt % or less.

In various aspects, the ratio of the components can also be adjusted to increase the antimicrobial activity or selectivity for a target microorganism. In various embodiments, the antibacterial botanical composition contains about 20 to 40 wt % *Origanum vulgare L.,* about 20 to 40 wt % *Thymus vulgaris L.,* about 10 to 20 wt % *Cinnamomum zeylanicum Nees,* about 10 to 30 wt % *Rosmarinum officinalis L.* and about 5 to 15 wt % *Lavandula officinalis L*. In certain aspects, the antimicrobial botanical active ingredients include about 20 wt % to about 40 wt % *Origanum vulgare L.,* about 20 wt % to about 40 wt % *Thymus vulgaris L*., about 10 wt % to about 30 wt % *Rosmarinum officinalis L*., and about 5 wt % to about 15 wt % *Lavandula officinalis L.* The botanical active ingredient mixture can also contain about 0.001 wt % to about 0.01 wt % *Hydrastis canadensis L.,* about 0.001 wt % to about 0.005 wt % olive leaf extract, and mixtures thereof. In further embodiments, the botanical active ingredients optionally include an effective amount of cinnamon bark extract (*Cinnamomum zeylanicum Nees*) to inhibit the growth of certain microbes.

In other aspects, at least one of the two botanical active ingredients is from *Curcuma longa* (tumeric) or *Cuminum cyminum* (cumin) and comprises tetrahydrocurcuminoid. It has been observed that such botanical active ingredients are highly efficacious when one or more vitamins is provided, such as vitamins like tocopherol (vitamin E), which will be described in more detail below. In certain aspects, the disclosure provides botanical active ingredients selected from *Romains Officinalis* (rosemary), *Origanum vulgare L* (oregano), and/or *Camellia sinensis* (tea). In certain aspects, such an oral composition further comprises a source of stannous ions.

In certain aspects, at least one of the two botanical active ingredients is from *Pinus Pinaster* (maritime pine) and comprises pycnogenol. In some variations, at least one of the two botanical active ingredients is obtained from *Petroselinum crispus* (parsley). Another suitable botanical active ingredient is from *Romains officinalis L.* (rosemary) and comprises carnosic acid. Another suitable botanical active ingredient is from *Salvadora persica* (miswak). In certain aspects, the botanical active ingredient is obtained from *Paullinia cupana* (guarana). In yet other aspects, the at least one botanical ingredient is selected from a plant of the genera *Calendula* or *Tagetes* (marigold). In certain aspects, at least one of the botanical active ingredients is from the genus *Boswellia* and comprises acetyl keto β-boswellic acid (AKBBA). Other suitable botanical active ingredients include those from *Copaifera langsdorfii,* or from *Sophora flavescens,* which comprises kurarinone. In various aspects, the botanical active ingredient optionally is obtained from *Euterpe oleracea* (Açaí palm tree). In certain aspects, at least one of the botanical active ingredients comprises rutin. Further, in certain embodiments, the oral composition further comprises a source of stannous ions in combination with rutin.

The botanical active ingredients may be selected from the following group: *Sambucus racemosa* (elderberry), *Origanum vulgare L* (oregano), and/or *Magnolia Officinalis* (magnolia), which have been found to be particularly efficacious. In other aspects, the botanical active ingredient is selected from *Scutellaria baicalensis* (baicalin), *Romains Officinalis* (rosemary), *Magnolia Officinalis* (magnolia), and/or *Camellia sinensis* (tea). Such mixtures of botanical ingredients have been shown to have particularly good efficacy at low concentrations for anti-microbial, antioxidant, anti-inflammatory, and anti-ageing oral composition, for example, at less than 4 parts per million (mg/kg).

In other embodiments, at least one of the botanical active ingredients is obtained from *Piper betle* (betel), *Syzygium aromaticum* (clove), *Commiphora myrrha* (myrrh), and/or *Juglans regia* (walnut). In various embodiments, the botanical active ingredient includes at least two compositions obtained from betel, clove, myrrh, and walnut.

The at least two botanical active ingredients can be present in the oral composition in various amounts and ratios, which depends upon the active ingredient employed and the concentration of active compounds contained therein. In various embodiments, each respective botanical active ingredient is present in the oral care composition about 0.001 to about 10% by weight of the total composition. In certain embodiments, a botanical active ingredient is present in the oral care composition about 0.01 to about 3%. In other embodiments, the botanical active ingredient is present at less than about 1%, for example the botanical active ingredient is present at a concentration of about 0.01 to about 1%. In one variation, the botanical active ingredient is present in the oral composition at a concentration of about 0.1 to about 0.3%.

In certain aspects, the total amount of botanical active ingredients present are at about 0.001% to about 20%; at about 0.01% to about 15%; at about 0.05% to about 10%; optionally at about 0.05% to about 5%; optionally at about 0.01% to about 1% by weight; and in certain aspects at about 0.1% to about 0.5%.

In certain embodiments, the oral compositions of the present disclosure optionally comprise one or more additional active ingredients which do not inhibit the efficacy of the botanical active ingredients previously described. The compositions of the present disclosure can comprise an optional active material, for example, a non-botanical active ingredient, which is operable for the prevention or treatment of a condition or disorder of hard or soft tissue of the oral cavity, the prevention or treatment of a physiological disorder or condition, or to provide a cosmetic benefit.

In various embodiments, the additional active is an "oral care active" operable to treat or prevent a disorder or provide a cosmetic benefit within the oral cavity (*e.g.*, to the teeth, gingiva or other hard or soft tissue of the oral cavity). Optional oral care actives among those useful herein include antibacterial agents, antiplaque agents, anti-adhesion, anti-oxidant, anticaries agents, anti-inflammatory agents, anti-ageing, densensitizing agents, whitening agents, tartar control agents, periodontal actives, nutrients, abrasives, breath freshening agents, malodour control agents, tooth desensitizers, salivary stimulants, and combinations thereof, such as those known to one of skill in the art. It is understood that while general attributes of each of the above categories of actives may differ, there may be some common attributes and any given material may serve multiple purposes within two or more of such categories of actives.

Compositions of the present disclosure may also be used for the treatment or prevention of systemic disorders, such as the improvement of overall systemic health characterized by a reduction in risk of development of systemic diseases, such as cardiovascular disease, stroke, diabetes, severe respiratory infection, premature and low birth weight infants (including associated post-partum dysfunction in neurological/developmental function), and associated increased risk of mortality.

In various aspects, such actives are selected for compatibility with the at least two botanical active ingredients, and with other ingredients of the composition to maintain a stable and efficacious oral composition, in other words, that the additional oral care actives do not detrimentally interfere with the activity or efficacy of the two or more botanical active ingredients described above. In certain aspects, the additional oral care active ingredients are nonionic and/or non-reactive with the at least two botanical active ingredients.

Examples of antibacterial phenolic compounds, including both synthesized and natural-based phenolic compounds, include 4-allylcatechol, *p*-hydroxybenzoic acid esters including benzylparaben, butylparaben, ethylparaben, methylparaben and propylparaben, 2-benzylphenol, butylated hydroxyanisole, butylated hydroxytoluene, capsaicin, carvacrol, creosol, eugenol, guaiacol, halogenated bisphenolics including hexachlorophene and bromochlorophene, 4-hexylresorcinol, 8-hydroxyquinoline and salts thereof, salicylic acid esters including menthyl salicylate, methyl salicylate and phenyl salicylate, phenol, pyrocatechol, salicylanilide, thymol, Triclosan (2', 4, 4'-trichloro-2-hydroxy-diphenyl ether) and Triclosan monophosphate.

The antibacterial phenolic compound is optionally present in a total amount of about 0.01% to about 10% by weight. Illustratively the total concentration of the at least one phenolic compound in a toothpaste or gel dentifrice or mouth rinse is optionally about 0.01 % to about 5%, for example about 0.1% to about 2%, about 0.2% to about 1% or about 0.25% to about 0.5%.

Other suitable antibacterial agents include, without limitation, copper (II) compounds such as copper (II) chloride, fluoride, sulfate and hydroxide, zinc ion sources such as zinc acetate, zinc citrate, zinc gluconate, zinc glycinate, zinc oxide, zinc sulfate and sodium zinc citrate, phthalic acid and salts thereof such as magnesium monopotassium phthalate, hexetidine, octenidine, sanguinarine, benzalkonium chloride, domiphen bromide, alkylpyridinium chlorides such as cetylpyridinium chloride (CPC) (including combinations of CPC with zinc and/or enzymes), tetradecylpyridinium chloride and N-tetradecyl-4-ethylpyridinium chloride, iodine, sulfonamides, bisbiguanides such as alexidine, chlorhexidine and chlorhexidine digluconate, piperidino derivatives such as delmopinol and octapinol, menthol, geraniol, citral, eucalyptol, antibiotics such as augmentin, amoxicillin, tetracycline, doxycycline, minocycline, metronidazole, neomycin, kanamycin and clindamycin, and the like. A further illustrative list of useful antibacterial agents is provided in U.S. Patent No. 5,776,435 to Gaffar et al., incorporated herein by reference. If present, these additional antimicrobial agents are present in an antimicrobial effective total amount, typically about 0.05% to about 10%, for example about 0.1% to about 3% by weight, of the composition.

Various optional oral care actives may be included in the oral composition of the present disclosure including those described above, such as antibacterial agents, antiplaque agents, anti-adhesion (that prevent adhesion of plaque to an enamel surface), anti-oxidant (such as Vitamin E or coenzyme Q10), anticaries agents, densensitizing agents (such as potassium citrate, potassium tartrate, potassium chloride, potassium sulfate and potassium nitrate), whitening agents (such as, urea peroxide, sodium percarbonate, sodium perborate and polyvinylpyrrolidone-H₂O₂); compatible enzymes; anti-inflammatory agents (such as, steroidal agents including flucinolone and hydrocortisone, and nonsteroidal agents (NTHEs)), tartar control agents, periodontal actives, chlorophyll compounds, nutrients (such as vitamins, minerals, and amino acids, lipotropics, fish oil, coenzymes and the like) abrasives, breath freshening/malodour control agents (such as α-ionone), and salivary stimulants (such as citric, lactic, malic, succinic, ascorbic, adipic, fumaric and tartaric acids); and any other suitable ingredients for oral care known to one of skill in the art. These additives, when present, are incorporated in the oral composition in amounts that do not substantially adversely affect the properties and characteristics desired, generally from concentrations of about 0.001 to about 10% by weight of the total composition.

In certain aspects, the oral composition having at least two botanical active ingredients further comprises a vitamin. Vitamins (or vitaminoids) useful herein can be natural or synthetic in origin and can be used in refined form or in crude form, for example as herbal/botanical preparations. Suitable vitamins can illustratively be selected from the following classes, many of which have been reported to possess antioxidant properties:
(a) sources of vitamin C, including ascorbic acid;
(b) 2-methyl-6-chromanol compounds, including TROLOX®, tocol (2-methyl-2-(4,8,12-trimethyltridecyl)-6-chromanol), α-tocopherol ((+)-2,5,7,8-tetramethyl-2-(4,8,12-trimethyltridecyl)-6-chromanol), β-tocopherol ((+)-2,5,8-trimethyl-2-(4,8,12-trimethyltridecyl)-6-chromanol), γ-tocopherol ((+)-2,7,8-trimethyl-2-(4,8,12-trimethyltridecyl)-6-chromanol), δ-tocopherol ((+)-8-methyl-2-(4,8,12-trimethyltridecyl)-6-chromanol), α-tocotrienol (2,5,7,8-tetramethyl-2-(4,8,12-trimethyl-3,7,11-tridecatrienyl)-6-chromanol), β-tocotrienol (2,5,8-trimethyl-2-(4,8,12-trimethyl-3,7,11-tridecatrienyl)-6-chromanol) and vitamin E (any one or a mixture of any two or more tocopherols and/or tocotrienols);
(c) carotenoids, including retinol (vitamin A), retinal, retinoic acid, α-carotene, β-carotene, γ-carotene, δ-carotene, lutein, lycopene, lycophyll, lycoxanthin, rhodoxanthin, astaxanthin and cryptoxanthin;
(d) sources of B vitamins, including thiamine (vitamin B₁), riboflavin (vitamin B₂), nicotinamide and nicotinic acid (both referred to as niacin), pantothenic acid (vitamin B₅), pantothenol, pyridoxine (vitamin B₆), pyridoxal, pyridoxamine, folic acid, dihydrofolic acid, vitamin B₁₂ and biotin;
(e) bioflavonoids, including rutin, hesperetin, hesperidin, eriodictyol, quercetin, quercetagetin and quercetagitrin;
(f) quinone-type enzyme cofactors, including ubiquinone (coenzyme Q₁₀) and pyrroloquinoline quinone (PQQ);
(g) sources of α-lipoic acid;
(h) sources of vitamin D, including calciferol and cholecalciferol; and
(i) orally acceptable salts, esters (including phosphate, acetate and long-chain, *e.g.*, linoleate and palmitate, esters), isomers, enantiomers, racemates and tautomers of the above.

One or more vitamins or vitaminoids are optionally present in a total amount of about 0.001% to about 10%, for example about 0.01% to about 5%; or about 0.1 % to about 3% by weight of the composition.

In another embodiment, the composition comprises an orally acceptable stannous ion source useful, for example, in helping reduce gingivitis, plaque, calculus, caries or sensitivity. One or more such sources can be present. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01 % to about 10%, for example about 0.1 % to about 7% or about 1% to about 5% by weight of the composition.

The oral composition of the present disclosure may contain an anticaries agent, such as a fluoride ion source or a fluorine-providing component. In various embodiments, the fluoride-based anticaries agent is present in an amount sufficient to supply about 25 ppm to 5,000 ppm of fluoride ions. Useful anticaries agents include inorganic fluoride salts, such as soluble alkali metal salts. For example, suitable fluoride sources useful in the oral composition are sodium fluoride, potassium fluoride, sodium fluorosilicate, ammonium fluorosilicate, sodium monfluorophosphate (MFP), and amine fluorides, including olaflur (N'-octadecyltrimethylendiamine-N,N,N'- tris(2-ethanol)-dihydrofluoride). The tin-based active ingredients described above are also suitable as anticaries agents. In certain embodiments, sodium fluoride and/or MFP are particularly suitable anticaries ingredients.

In various embodiments, the oral compositions of the present disclosure comprise antitartar agents to prevent and/or minimize calculus formation. One or more of such agents can be present. Suitable anticalculus agents include without limitation: phosphates and polyphosphates. Phosphate and polyphosphate salts are generally employed in the form of their wholly or partially neutralized water soluble cationic species (e.g., potassium, sodium or ammonium salts, and any mixtures thereof). Thus, useful inorganic phosphate and polyphosphate salts illustratively include monovalent cations with monobasic, dibasic and tribasic phosphates; tripolyphosphate and tetrapolyphosphate; mono-, di-, tri- and tetra-pyrophosphates; and cyclophosphates (also generally known in the art as "metaphosphates"). Useful monovalent cations of such phosphate salts include hydrogen, monovalent metals including alkali metals, and ammonium, for example.

Examples of useful antitartar agents include Na₅P₃O₁₀ (sodium tripolyphosphate or STPP), tetraalkali metal pyrophosphate salts such as Na₄P₂O₇ (tetrasodium pyrophosphate or TSPP), K₄P₂O₇ (tetrapotassium pyrophosphate), Na₂K₂P₂O₇ (disodium dipotassium pyrophosphate), Na₂H₂P₂O₇ (disodium dihydrogen pyrophosphate) and K₂H₂P₂O₇ (dipotassium dihydrogen pyrophosphate). Cyclophosphates, which are generally referred to as "metaphosphates", are cyclic phosphate anion compounds. Those useful as tartar control agents include, sodium hexametaphosphate and sodium trimetaphosphate, for example. In one embodiment, the anticalculus system comprises sodium tripolyphosphate (STPP) and/or tetrasodium pyrophosphate (TSPP).

Other suitable tartar control agents include polyaminopropanesulfonic acid (AMPS), zinc citrate trihydrate, polypeptides such as polyaspartic and polyglutamic acids, polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and salts of any of these agents, for example their alkali metal and ammonium salts.

In various embodiments where the anticalculus/anti-tartar active ingredients are present in the oral compositions, they range in concentration at about 0.01 to about 10% by weight, optionally at about 1 to about 5 % by weight.

Additionally, various embodiments of the present disclosure include an anticalculus system that further comprises a synthetic anionic linear polycarboxylate polymer. The anionic linear polycarboxylate is generally synthesized by using an olefinically or ethylenically unsaturated carboxylic acid that contains an activated carbon-to-carbon olefinic double bond and at least one carboxyl group. The acid contains an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrilacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other olefinic monomers copolymerizable with such carboxylic monomers include vinyl acetate, vinyl chloride, dimethyl maleate and the like. The synthetic anionic linear polymeric polycarboxylate component is mainly a hydrocarbon with optional halogen and O-containing substituents and linkages as present in for example ester, ether and OH groups. The copolymers preferably contain sufficient carboxylic salt groups for water-solubility. The terms "synthetic" and "linear" do not include known thickening or gelling agents comprising carboxymethylcellulose and other derivatives of cellulose and natural gums, nor Carbopols having reduced solubility due to cross-linkages.

In certain aspects, copolymers of maleic anhydride or acid with another polymerizable ethylenically unsaturated monomer are 1:4 to 4:1 copolymers, preferably methyl vinyl ether (methoxyethylene) having a molecular weight (M.W.) of about 30,000 to about 2,500,000. These copolymers are commercially available, for example as GANTREZ^{™}AN-139 (M.W. 1,100,000), AN-119 (M.W. 200,000) and S-97 Solution (M.W. 1,500,000), from ISP Corporation.

In various embodiments, where the anti-tartar/anticalculus system comprises a synthetic anionic polycarboxylate, it is preferably present in an amount of about 0.1 to about 5 weight %. In another embodiment, the synthetic anionic polycarboxylate is present in an amount of about 0.5 to about 1.5 weight %, most preferably at about 1 weight % of the oral care composition. In one embodiment according to the present disclosure, the anticalculus system comprises a copolymer of maleic anhydride and methyl vinyl ether, such as for example, the GANTREZ^{™} S-97 product discussed above. In one embodiment, the antitartar active ingredient system of the oral care composition comprises TSPP at about 0.5 to about 1.5% by weight, STPP at about 1 to about 10% by weight, and a copolymer of maleic anhydride and methyl vinyl ether at about 0.5 to about 1.5 % by weight.

In certain aspects, the oral composition includes a "bioavailability-enhancing agent," which refers to one or more constituents that are present in the oral composition that improve the degree to which at least one of the oral active ingredients or other substance become available to the target tissue after administration to the oral cavity. In various embodiments, the bioavailability-enhancing agent improves the availability of at least one of the botanical active ingredients to the target oral tissue. In certain aspects, the bioavailability-enhancing agent improves the availability of all of the botanical active ingredients to oral surfaces. The botanical active ingredients tend to be lipophilic and the bioavailability-enhancing agent enhances tissue uptake and/or efficacy of the active at the oral surface, even in the relatively aqueous environment of the oral cavity. In certain aspects, the bioavailability-enhancing agent is at least one of a solubilizing agent and an efficacy-enhancing agent.

In certain embodiments, the bioavailability-enhancing agent comprises an agent that comprises water soluble or swellable anionic polymer or copolymer having a group or moiety that enhances delivery of the botanical active ingredients to a subject's oral tissue. The bioavailability-enhancing agent is a polymer or copolymer, which terms are entirely generic, thus including for example, oligomers, homopolymers, copolymers of two or more monomers, ionomers, block copolymers, graft copolymers, cross-linked polymers and copolymers, and the like. They may be natural or synthetic, and water (saliva) soluble or swellable (hydratable, hydrogel forming) polymer or copolymer.

If the efficacy-enhancing agent comprises a delivery-enhancing group, it can be any of those listed in U.S. Pat. Nos. 5,538,715 and 5,776,435, both to Gaffar et al. In various embodiments, a delivery-enhancing moiety of the bioavailability-enhancing agent is acidic such as sulfonic, phosphinic, or more preferably phosphonic or carboxylic, or a salt thereof, *e.g.,* alkali metal or ammonium. When present, a retention-enhancing group(s) on the bioavailability-enhancing agent is optionally any organic retention-enhancing group, for example, those that have the formula --(X)ₙ--R wherein X is O, N, S, SO, SO₂, P, PO or Si or the like, R is hydrophobic alkyl, alkenyl, acyl, aryl, alkaryl, aralkyl, heterocyclic or their inert-substituted derivatives, and n is zero or one or more. The term "inert-substituted derivatives" is intended to include substituents on R which are generally non-hydrophilic and do not significantly interfere with the desired functions of the bioavailability-enhancing agent as enhancing the delivery of the botanical active ingredients to, and retention thereof, on oral surfaces such as halogen, *e.g.*, Cl, Br, I, and carboxy and the like.

Synthetic anionic polycarboxylates may also be used in the dentifrice compositions of the present disclosure as a bioavailability/efficacy-enhancing agent for certain active ingredients, including botanical active ingredients (as discussed above) or other active agents within the oral composition. Such anionic polycarboxylates are generally employed in the form of their free acids or preferably partially or more preferably fully neutralized water soluble alkali metal (*e.g.*, potassium and preferably sodium) or ammonium salts. As discussed above, suitable copolymers are of maleic anhydride or acid with another polymerizable ethylenically unsaturated monomer, preferably methylvinylether/maleic anhydride having an approximate molecular weight (M.W.) of about 30,000 to about 2,500,000 most preferably about 30,000 to about 2,000,000. Examples of these copolymers are available from ISP Corporation under the tradename GANTREZ^{™}, e.g. AN 139 (M.W. 1,100,000), AN 119 (M.W. 200,000); S-97 Pharmaceutical Grade (M.W. 1,500,000), AN 169 (M.W. 2,000,000), and AN 179 (M.W. 2,400,000); wherein a particularly suitable copolymer is S-97 Pharmaceutical Grade (M.W. 1,500,000).

The anionic polycarboxylate, is employed in certain embodiments in amounts effective to achieve the desired enhancement of the efficacy of any antibacterial, antitartar or other active agent within the dentifrice or other oral composition. Generally, the anionic polycarboxylates is present within the dentifrice composition about 0.05% to about 5% by weight, preferably about 0.5% to about 2.5% by weight.

In various embodiments of the present disclosure, the oral composition comprises an orally acceptable carrier in addition to the two or more botanical active ingredients. As used herein, an "orally acceptable carrier" refers to a material or combination of materials that are safe for use in the compositions of the present disclosure, commensurate with a reasonable benefit/risk ratio, with which the botanical active ingredients may be associated while retaining significant efficacy. The orally acceptable carrier may comprise and be compatible with a variety of other conventional active ingredients known to one of skill in the art, including, tartar control agents, antibacterial agents, anticaries agents, sensitivity agents, and the like. In certain aspects, the components of the carrier are specifically selected to ensure that there is substantially no reduction of the efficacy or bioavailability of the botanical active ingredients.

A suitable vehicle or carrier includes one or more compatible solid or liquid fillers, diluents, excipients, or encapsulating substances, which are suitable for topical administration to oral tissue surfaces. In various aspects, the orally acceptable carrier does not cause irritation, swelling or pain and does not typically produce an allergic or untoward reaction such as gastric upset, nausea or dizziness. Selection of specific carrier components is dependant on the desired product form, including dentifrices, toothpastes, tooth powders, prophylaxis pastes, dental floss, mouth rinses, lozenges, gums, beads, gels, paints, animal products, and the like. Such carriers are well known to those of skill in the art; however, certain exemplary vehicles will be discussed herein.

In various embodiments, the orally acceptable dentifrice carrier used to prepare an oral composition comprises a water-phase. As recognized by one of skill in the art, the oral compositions of the present disclosure optionally include other materials, such as for example, viscosity modifiers, diluents, surface active agents, such as surfactants, emulsifiers, and foam modulators, pH modifying agents, abrasives, humectants, mouth feel agents, sweetening agents, flavor agents, colorants, preservatives and combinations thereof. It is understood that while general attributes of each of the above categories of materials may differ, there may be some common attributes and any given material may serve multiple purposes within two or more of such categories of materials. Preferably, such other carrier materials are also selected for compatibility with the botanical active ingredients, as well as with other ingredients of the composition.

The term "mouthrinse" in the present disclosure refers to oral compositions that are substantially liquid in character, such as a mouthwash, spray, or rinse. In such a preparation the orally acceptable carrier typically has an aqueous phase comprising water or a water and alcohol mixture. Further, in various embodiments, the oral carrier has a humectant and surfactant as described below. Generally, the weight ratio of water to alcohol is in the range of about 1:1 to about 20:1, preferably about 3:1 to 10:1 1 and more preferably about 4:1 1 to about 6:1. The total amount of water-alcohol mixture in this type of preparation is typically in the range of about 70 to about 99.9% of the preparation. In various embodiments, the alcohol is typically ethanol or isopropanol.

The pH of such liquid and other preparations of the disclosure is generally in the range of about 4.5 to about 10. The pH can be controlled with acid (*e.g.*, citric acid or benzoic acid) or base (*e.g.*, sodium hydroxide) or buffered (with sodium citrate, benzoate, carbonate, or bicarbonate, disodium hydrogen phosphate, or sodium dihydrogen phosphate, for example).

In various embodiments, the aqueous oral composition (*e.g.*, mouthrinse) contains a humectant and a surfactant. The humectant is generally a mixture of humectants, such as glycerin and sorbitol, and a polyhydric alcohol such as propylene glycol, butylene glycol, hexylene glycol, polyethylene glycol. The humectant content is in the range of about 5 to about 40% and preferably about 10 to about 30%. Surfactants useful in the present embodiment include anionic, nonionic, and zwitterionic surfactants. The surfactant is present in the aqueous oral compositions of the present disclosure range about 0.01% to about 5%, preferably about 0.5% to about 2.5%.

The term "confectionery composition" as used herein includes chewing gums, and orally soluble tablets, beads and lozenges. Saliva dissolves the lozenge or chewable gum product, and promotes prolonged contact with oral surfaces so that the delivery of the active ingredient in a lozenge tablet, bead or chewing gum form ensures that an adequate dosage of the active ingredients are delivered to the oral surface when the product is used.

In the present embodiment, the orally acceptable carrier is in the form of a lozenge, bead, tablet or chewing gum or other similar solid delivery system. Such delivery systems are well known to one of skill in the art, and generally entail stirring the active anti-oxidant agent into a warm base with flavor, and non-cariogenic sweeteners.

The orally acceptable vehicle or carrier in a lozenge bead or tablet is a non-cariogenic, solid water-soluble polyhydric alcohol (polyol) such as mannitol, xylitol, sorbitol, malitol, hydrogenated starch hydrozylate, hydrogenated glucose, hydrogenated disaccharides or hydrogenated polysaccharides, in an amount of about 85 to about 95% of the total composition. Emulsifiers such as glycerin, and tableting lubricants, in minor amounts of about 0.1 to 5%, may be incorporated into the tablet, bead or lozenge formulation to facilitate the preparation of the tablet beads and lozenges. Suitable lubricants include vegetable oils such as coconut oil, magnesium stearate, aluminum stearate, talc, starch and Carbowax. Suitable non-cariogenic gums include kappa carrageenan, carboxymethyl cellulose, hydroxyethyl cellulose and the like.

The lozenge, bead or tablet may optionally be coated with a coating material such as waxes, shellac, carboxymethyl cellulose, polyethylene/maleic anhydride copolymer or kappacarrageenan to further increase the time it takes the tablet or lozenge to dissolve in the mouth. The uncoated tablet or lozenge is slow dissolving, providing a sustained release rate of active ingredients of about 3 to 5 minutes. Accordingly, the solid dose tablet, bead and lozenge compositions of this embodiment afford a relatively longer time period of contact of the teeth in the oral cavity with the botanical active ingredients of the present disclosure.

The chewing gum of the present disclosure is preferably a sugarless chewing gum containing the antioxidant compound(s). Chewing gum formulations typically contain, in addition to a chewing gum base, one or more plasticizing agents, at least one sweetening agent and at least one flavoring agent.

Gum base materials suitable for use in the practice of this disclosure are well known in the art and include natural or synthetic gum bases or mixtures thereof. Representative natural gums or elastomers include chicle, natural rubber, jelutong, balata, guttapercha, lechi caspi, sorva, guttakay, crown gum, perillo, or mixtures thereof. Representative synthetic gums or elastomers include butadiene-styrene copolymers, polyisobutylene and isobutylene-isoprene copolymers. The gum base is incorporated in the chewing gum product at a concentration of about 10 to about 40% and preferably about 20 to about 35%.

Plasticizing/softening agents commonly used in chewing gum compositions are suitable for use in this disclosure, including gelatin, waxes and mixtures thereof in amounts of about 0.1 to about 5%. The sweetening agent ingredient used in the practice of this disclosure may be selected from a wide range of materials, and include the same artificial and polyol sweeteners used for the preparation of tablets, beads and lozenges. Polyol sweeteners such as sorbitol and malitol are present in the chewing gum composition of the present disclosure in amounts of about 40 to about 80% and preferably about 50 to about 75%. The artificial sweetener is present in the chewing gum composition of the present disclosure in amounts of about 0.1 to about 2% and preferably about 0.3 to about 1%.

In certain other desirable forms of this disclosure, the oral composition may be a dentifrice. As referred to herein, a "dentifrice" is a composition that is intended for cleaning an oral surface (hard or soft) within the oral cavity. Such dentifrices include toothpowder, a dental tablet, toothpaste (dental cream), or gel. In a toothpaste dentifrice, the orally acceptable carrier may comprise water and humectant typically in an amount of about 10% to about 80% of the oral composition.

In various embodiments of the present disclosure, glycerin, propylene glycol, sorbitol, polypropylene glycol and/or polyethylene glycol (*e.g.*, 400-600) are suitable humectants/carriers. Also advantageous are liquid mixtures of water, glycerin and sorbitol. In certain embodiments where the carrier is a clear gel and where the refractive index is an important consideration, the composition comprises about 3 to about 30% of water, 0 to about 70% of glycerin and about 20-80% of sorbitol.

In various embodiments, such as for toothpastes, creams and gels, the oral composition contains a natural or synthetic thickener or gelling agent, which, other than silica thickeners, include natural and synthetic gums and colloids. Such suitable thickeners include naturally occurring polymers such as carrageenans, xanthan gum, synthetic thickener such as polyglycols of varying molecular weights sold under the tradename Polyox and cellulose polymers such as hydroxyethyl cellulose and hydroxpropyl cellulose. Other inorganic thickeners include natural and synthetic clays such as hectorite clays, lithium magnesium silicate (laponite) and magnesium aluminum silicate (Veegum). Other suitable thickeners are synthetic hectorite, a synthetic colloidal magnesium alkali metal silicate complex clay available for example as LAPONITE^{™} (*e.g.*, CP, SP 2002, or D) marketed by Laporte Industries Limited. LAPONITE^{™} D analysis shows, approximately, 58% SiO₂, 25.4% MgO, 3.05% Na₂O, 0.98% Li₂O, and some water and trace metals, and has a true specific gravity of 2.53 and an apparent bulk density (g/mL at 8% moisture) of 1.0. In certain embodiments, the thickening agent is present in the dentifrice composition in amounts of about 0.1 to about 10%, preferably about 0.5 to about 5.0%.

Other suitable thickeners include Irish moss, gum tragacanth, starch, polyvinylpyrrolidone, hydroxyethyl propyl cellulose, hydroxybutyl methyl cellulose, hydroxypropyl methyl cellulose, hydroxyethyl cellulose (e.g. available as NATROSOL^{™}), sodium carboxymethyl cellulose, and colloidal silica such as finely ground Syloid (e.g. 244).

Various embodiments of the present disclosure also comprise a surface active agent, which may function as a surfactant, emulsifier, and/or foam modulator. Surface active agents generally achieve increased prophylactic action, by thoroughly dispersing the active ingredients throughout the oral cavity. Suitable emulsifying agents are those which are reasonably stable and foam throughout a wide pH range, including non-soap anionic, nonionic, zwitterionic and amphoteric organic synthetic detergents. Further, surface active ingredients preferably render the instant compositions more cosmetically acceptable. The organic surface-active material is preferably anionic, nonionic or ampholytic in nature, and preferably a detersive material which imparts to the composition detersive and foaming properties. In certain embodiments, one or more surfactants are present in the oral composition of the present disclosure at about 0.001% to about 5%, optionally at about 0.5% to about 2.5%.

Nonionic surfactants useful in the compositions of the present disclosure include compounds produced by the condensation of alkylene oxides (especially ethylene oxide) with an organic hydrophobic compound, which may be aliphatic or alkylaromatic in nature. One group of surfactants is known as "ethoxamers" - they are condensation products of ethylene oxide with fatty acids, fatty alcohols, fatty amides, polyhydric alcohols, (e.g., sorbitan monostearate) and the like. "Polysorbates" is the name given to a class of nonionic surfactants prepared by ethoxylating the free hydroxyls of sorbitan-fatty acid esters. They are commercially available, for example as the TWEEN^{®} surfactants of ICI. Non-limiting examples include Polysorbate 20 (polyoxyethylene 20 sorbitan monolaurate, TWEEN^{®} 20) and Polysorbate 80 (polyoxyethylene 20 sorbitan mono-oleate, TWEEN^{®} 80). Particularly suitable polysorbates include those with about 20 to 60 moles of ethylene oxide per mole of sorbitan ester.

Other suitable nonionic surfactants include poly(oxyethylene)-poly(oxypropylene) block copolymers, especially triblock polymers of this type with two blocks of poly(oxyethylene) and one block of poly(oxypropylene). Such copolymers are known commercially by the non-proprietary name of poloxamers, the name being used in conjunction with a numeric suffix to designate the individual identification of each copolymer. Poloxamers may have varying contents of ethylene oxide and propylene oxide, leading to a wide range of chemical structures and molecular weights. One particularly suitable poloxamer is Poloxamer 407. It is widely available, for example under the tradename PLURONIC^{®} F127 of BASF Corporation.

Other non-limiting examples of suitable nonionic surfactants include products derived from the condensation of ethylene oxide with the reaction product of propylene oxide and ethylene diamine, long chain tertiary amine oxides, long chain tertiary phosphine oxides, long chain dialkyl sulfoxides and the like.

Other surfactants useful in various embodiments of the present disclosure include zwitterionic synthetic surfactants. Certain of these can be broadly described as derivatives of aliphatic quaternary ammonium, phosphonium, and sulfonium compounds, in which the aliphatic radicals can be straight chain or branched, and where one of the aliphatic substituents contains from 8 to 18 carbon atoms and one contains an anionic water-solubilizing group, e.g., carboxy, sulfonate, sulfate, phosphate or phosphonate. One example of a suitable zwitterionic surfactant is 4-(N,N-di(2-hydroxyethyl)-N-octadecylammonio)-butane-1-carboxylate.

Other suitable zwitterionic surfactants include betaine surfactants, such as those disclosed in U.S. Patent 5,180,577. Typical alkyldimethyl betaines include decyl betaine 2-(N-decyl-N,N-dimethylammonio) acetate, cocobetaine, myristyl betaine, palmityl betaine, lauryl betaine, cetyl betaine, stearyl betaine, and the like. The amidobetaines are exemplified by cocoamidoethyl betaine, cocoamidopropyl betaine, lauramidopropyl betaine and the like. Particularly useful betaine surfactants include cocoamidopropyl betaine and lauramido propyl betaine.

Suitable examples of anionic surfactants are water-soluble salts of higher fatty acid monoglyceride monosulfates, such as the sodium salt of the monosulfated monoglyceride of hydrogenated coconut oil fatty acids, higher alkyl sulfates such as sodium lauryl sulfate (SLS), alkyl aryl sulfonates such as sodium dodecyl benzene sulfonate, higher alkyl sulfoacetates, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine which are preferably substantially free from soap or similar higher fatty acid material.

In various embodiments of the present disclosure, where the carrier of the oral care composition is solid or a paste, the oral composition preferably comprises a dentally acceptable abrasive material, which may serve to either polish the tooth enamel or provide a whitening effect.

In the preparation of a dentifrice composition, abrasives, which may be used in the practice of the present disclosure, include silica abrasives such as precipitated silicas having a mean particle size of up to about 20 microns, such as ZEODENT^{™} 115, marketed by J. M. Huber. One useful abrasive is marketed under the trade designation ZEODENT^{™} 105 by J. M Huber Co, which has a low abrasiveness to tooth enamel, and is a precipitated silica that is about 7 to about 10 microns in diameter, has a BET surface area of 390 m²/g of silica, and an oil absorption of less than 70 cm³/100 g of silica. Other useful dentifrice abrasives include sodium metaphosphate, potassium metaphosphate, tricalcium phosphate, dihydrated dicalcium phosphate, anhydrous dicalcium phosphate, aluminum silicate, calcined alumina, bentonite or other siliceous materials, or combinations thereof.

In other embodiments of the present disclosure, useful abrasive materials for preparing dentifrice compositions include silica gels and precipitated amorphous silica having an oil absorption value of less than 100 cm³/100 g silica and preferably in the range of about 45 cm³/100 g to less than about 70 cm³/100 g silica. Oil absorption values are measured using the ASTA Rub-Out Method D281. These are colloidal particles of silica having an average particle size ranging about 3 microns to about 12 microns, and more preferably between about 5 to about 10 microns and a pH range from 4 to 10, preferably 6 to 9 when measured as a 5% slurry.

One useful abrasive is marketed under the trade designation ZEODENT^{™} 105 by J. M Huber Co, which has a low abrasiveness to tooth enamel, and is a precipitated silica that is about 7 to about 10 microns in diameter, has a BET surface area of 390 m²/g of silica, and an oil absorption of less than 70 cm³/100 g of silica. Further suitable abrasives useful with various embodiments of the present disclosure are low oil of absorption silica abrasives such as those marketed under the trade designation SYLODENT^{™} XWA or SYLODENT^{™} 783 by Davison Chemical Division of W. R. Grace & Co., Baltimore, Md. 21203. SYLODENT^{™} t XWA 650, a silica hydrogel composed of particles of colloidal silica having a water content of 29% averaging about 7 to about 10 microns in diameter, and an oil absorption of less than 70 cm³/100 g of silica is an example of a low oil absorption silica abrasive useful in the practice of the present disclosure. The abrasive is present in the dentifrice composition of the present disclosure at a concentration of about 10 to about 40% and preferably about 15 to about 30%.

Other suitable polishing materials include the particulate thermosetting resins, such as melamine, phenolic, and urea-formaldehydes, and cross-linked polyepoxides and polyesters. Suitable polishing materials include crystalline silica having particle sizes of up to about 5 microns, a mean particle size of up to about 1 µm, and a surface area of up to about 50,000 cm²/g, silica gel or colloidal silica, and complex amorphous alkali metal aluminosilicate.

In certain aspects, suitable abrasives in accordance with certain embodiments of the present disclosure comprise dihydrated dicalcium phosphate, anhydrous dicalcium phosphate, precipitated calcium carbonate (PCC) or combinations thereof.

In embodiments where the dentifrice is a clear or transparent gel, a polishing agent of colloidal silica, such as those sold under the trademark SYLOID^{™} as Syloid 72 and Syloid 74 or under the trademark SANTOCEL^{™} as Santocel 100 alkali metal almuino-silicate complexes are particularly useful, since they have refractive indices close to the refractive indices of gelling agent-liquid (including water and/or humectant) systems commonly used in dentifrices.

Many of the so-called "water-insoluble" polishing materials are anionic in character and also include small amounts of soluble material. Thus, insoluble sodium metaphosphate, known as Madrell's salt and Kurrol's salt, are examples of suitable polishing materials. These metaphosphate salts exhibit only a minute solubility in water, and therefore are commonly referred to as insoluble metaphosphates (IMP). Such IMPs generally contain a minor amount, usually a few percent (e.g., < 4%), of soluble phosphate material as impurities. Some of these impurities can be removed by pre-washing the material. In certain aspects, the insoluble alkali metal metaphosphate is typically employed in powder form of a particle size such that no more than 1% of the material is larger than about 37 µm.

In certain embodiments, the abrasives may also include whiteness-imparting abrasive particles which include for example, a metal oxide. The metal oxide can comprise any metal oxide that provides a white color, such as, for example, titanium oxide, aluminum oxide, tin oxide, calcium oxide, magnesium oxide, barium oxide, or a combination thereof. Certain whiteness imparting abrasives are also pearlescent particles, which comprise a single mineral or chemical species, such as, for example a silicate such as mica, or bismuth oxychloride. By "mica" it is meant any one of a group of hydrous aluminum silicate minerals with platy morphology and perfect basal (micaceous) cleavage. Mica can be, for example, sheet mica, scrap mica or flake mica, as exemplified by muscovite, biotite or phlogopite type micas. In some embodiments, the pearlescent particles can comprise a complex comprising more than one mineral or chemical species, such as, for example, mica coated with a metal oxide such as titanium oxide.

In embodiments where the dentrifrice is in a solid or paste form, the abrasive material is generally present at about 10% to about 99% of the oral composition. In certain embodiments, the polishing material is present in amounts ranging about 10% to about 75% in toothpaste, and about 70% to about 99% in toothpowder.

In various embodiments of the present disclosure, water is also present in the oral composition, as referred to above. Water employed in the preparation of commercially suitable toothpastes, gels, and mouthwashes should preferably be deionized, ultraviolet treated, and free of organic impurities. Water generally comprises about 10% to 50%, preferably about 20% to 40%, of the toothpaste compositions herein. The water is free water which is added, plus that which is introduced with other materials for example, such as that added with sorbitol.

In various embodiments, the oral care composition of the present disclosure contains a flavoring agent. Such flavoring agents may not be necessary depending on the selection of the two or more botanical active ingredients, which may provide suitable flavoring. Conventional flavoring agents include essential oils as well as various flavoring aldehydes, esters, alcohols, and similar materials. Any suitable flavoring or sweetening material may also be employed. Examples of suitable flavoring constituents are flavoring oils, *e.g.*, oil of spearmint, pepperment, wintergreen, sassafras, clove, sage, eucalyptus, marjoram, cinnamon, lemon, lime, orange, grapefruit, and methyl salicylate. Also useful are such chemicals as menthol, carvone, and anethole. Suitable sweetening agents include sucrose, lactose, maltose, sorbitol, xylitol, sodium cyclamate, perillartine, AMP (aspartyl phenyl alanine, methyl ester), saccharine and the like. The flavor and sweetening agents may each or together be incorporated into the oral composition at a concentration of about 0.001 to about 5% and preferably about 0.5 to about 2%.

## Claims

1. A composition comprising:
at least two botanical active ingredients derived from one or more plants of the following genera: *Origanum, Thymus, Lavandula, Salvia, Melissa, Cuminum, Petroselinum, Calendula, Tagetes, Boswellia, Sambucus, Copaifera, Curcuma, Allium, Symphytum, Punica, Euterpe, Sophora, Rheum, Fagopyrum, Camellia, Coptis, Hydrastis, Mahonia, Phellodendron, Berberis, Xanthorhiza, Lonicera, Vaccinium, Cinnamomum, Vitus, Terminalia, Pinus, Albizia, Melia, Salvadora, Paullinia, Piper, Syzygium, Commiphora, Juglans, Scutellaria,* and *Magnolia*;
and an orally acceptable vehicle to deliver an effective amount of the at least two active ingredients *in vivo,*
wherein
(a) at least one of the two botanical active ingredients is derived from one or more plants of the following species: *Salvia officinalis, Salvia divinorum, Salvia apiana, Melissa Officinalis, Cuminum cyminum, Petroselinum crispum, Calendula arvensis, Calendula maderensis, Calendula officinalis, Tagetes erecta, Tagetes minuta, Tagetes patula, Boswellia sacra, Boswellia frereana, Boswellia serrata, Boswellia papyrifera, Sambucus nigra, Sambucus melanocarpa, Sambucus racemosa, Copaifera langsdorfii, Curcuma longa, Allium sativu, Symphytum officinale, Punica granatum, Euterpe oleracea, Sophora flavescens, Rheum rhabarbarum, Rheum rhaponticum, Fagopyrum esculentum, Coptis teeta, Hydrastis canadensis, Mahonia aquifolium, Phellodendron amurense, Berberis vulgaris, Xanthorhiza simplicissima, Lonicera ceprifoliu, Vitis Vinifera, Terminalia Bellerica, Pinus Pinaster, Albizia Lebbek, Melia Azadirachta, Salvadora persica, Paullinia cupana, Piper betle, Syzygium aromaticum, Commiphora myrrha,* and *Juglans regia*;
or
(b) the at least two botanical active ingredients are derived from one or more plants of the following species: *Romains officinalis, Origanum vulgare L, Thymus vulgaris L, Lavandula angustifolia*/*officinalis, Salvia officinalis, Melissa officinalis, Cuminum cyminum, Petroselinum crispum, Calendula officinalis, Tagetes erecta, Boswellia sacra, Sambucus nigra, Copaifera langsdorfii, Curcuma longa, Allium sativu, Symphytum officinale, Punica granatum, Euterpe oleracea, Sophora flaveseens, Rheum rhabarbarum, Fagopyrum esculentum, Camellia sinensis, Coptis teeta, Hydrastis canadensis, Mahonia aquifolium, Phellodendron amurense, Berberis vulgaris, Xanthorhiza simplicissima, Lonicera ceprifoliu, Vaccinium macrocarpon, Cinnamomum zeylanicum Nees, Cinnamomum verum, Vitis Vinifera, Terminalia Bellerica, Pinus Pinaster, Albizia Lebbek, Melia Azadirachta, Salvadora persica, Paullinia cupana, Piper betle, Syzygium aromaticum, Commiphora myrrha, Juglans regia, Scutellaria baicalensis,* and *Magnolia officinalis.*

2. The composition of claim 1 wherein at least one of the two botanical active ingredients is from *Curcuma longa* and/or *Cuminum cyminum* and comprises tetrahydrocurcuminoid.

3. The composition of claim 2 wherein the oral composition further comprises tocopherol (vitamin E).

4. The composition of claim 1 wherein at least one of the two botanical active ingredients is derived from *Pinus Pinaster* and comprises pycnogenol.

5. The composition of claim 1 wherein at least one of the botanical active ingredients is derived from *Sophora flavescens* and comprises kurarinone.

6. The composition of claim 1 wherein at least one of the botanical active ingredients is derived from *Fagopyrum esculentum.*

7. The composition of claim 1 wherein at least one of the botanical active ingredients is derived from *Euterpe oleracea.*

8. The composition of claim 1 wherein, in (a), at least one of the two botanical active ingredients is derived from *Calendula arvensis, Calendula maderensis, Calendula officinalis, Tagetes erecta, Tagetes minuta* or *Tagetes patula.*

9. The composition of claim 1 wherein, in (a), at least one of the two botanical active ingredients is derived from *Sambucus nigra, Sambucus melanocarpa,* and *Sambucus racemosa.*

10. The composition of claim 1 wherein, in (b), at least one of the botanical active ingredients is derived from *Romains officinalis L.* and comprises carnosic acid.

11. The composition of claim 1 wherein, in (b), the botanical active ingredients comprise an active ingredient derived from each of the plants: *Origanum vulgare L; Thymus vulgaris L; Cinnamonmum zeylanicum Nees; Romains officinalis L*., *Lavandula angustifolia*/*officinalis L.* and *Hydrastis canadaensis L.;* and optionally further comprise an additional botanical active ingredient derived from *Cinnamonmum zeylanicum Nees*

12. The composition of any one of claims 1-11 further comprising an orally acceptable stannous ion source.

13. The composition of any one of claims 1-11 wherein the oral composition comprises one or more additional oral active ingredients selected from the group consisting of anti-tartar agents, antibacterial agents, anti-inflammatory agents, anticaries agents, whitening agents, densensitizing agents, vitamins, compatible enzymes, chlorophyll compounds, periodontal actives, breath freshening agents, malodour control agents, salivary stimulants and combinations thereof; or wherein the orally acceptable vehicle comprises one or more components selected from the group consisting of viscosity modifiers, diluents, surface active agents, pH modifying agents, abrasives, humectants, mouth feel agents, sweetening agents, flavor agents, colorants, preservatives, and combinations thereof.

14. The composition of claim 1, wherein a total amount of the at least two botanical active ingredients is 0.001 to 5% by weight of the total oral composition.

15. The composition of claim 1 for use in a method of promoting and/or maintaining systemic health by topically applying the oral composition to the surfaces of the oral cavity.
